# EUROPEAN PATENT APPLICATION

(11) **EP 3 473 268 A1**
(43) Date of publication of application: **24.04.2019**
(21) Application number: 17197552.7
(22) Date of filing: 20.10.2017
(51) Int. Cl.: A61K 39/02

(54) **COMBINED PREPARATION FOR PARENTERAL USE**

(71) Applicant: IDT Biologika GmbH, 06861 Dessau-Rosslau (DE)
(72) Inventor: SELBITZ, Hans-Joachim, 06861 Dessau-Rosslau (DE); FLORIAN, Volker, 06861 Dessau-Rosslau (DE); LÜDER, Olaf, 06861 Dessau-Rosslau (DE)
(74) Representative: Isarpatent

(57) **Abstract**

The present invention is directed to a combined preparation for parenteral use comprising an adsorbed vaccine and complexed iron in a physiologically acceptable form. The present invention is further directed to the use of such a combined preparation in the immunization of animals such as piglets.

## Description

The present invention is directed to a combined preparation for parenteral use comprising an adsorbed vaccine and complexed iron in a physiologically acceptable form. The present invention is further directed to the use of such a combined preparation in the immunization of animals such as piglets.

### BACKGROUND OF THE INVENTION

Vaccination of farm animals, such as pigs and piglets, can help protect the animals from many common diseases such as foot-and-mouth disease, swine fever, swine influenza as examples for major virus diseases or diseases caused by bacteria such as E. *coli*-caused diseases, leptospirosis, Glässer's disease and many others. Vaccines commonly used on pig farms include those against erysipelas, parvovirus infection (SMEDI syndrome), *E. coli* diarrhoea, enzootic pneumonia caused by *Mycoplasma hyopneumoniae, Streptococcus suis* infections, *Haemophilus parasuis* infections, *Brachyspira hyodysenteriae* caused infections, necrotic pleuropneumonia caused by *Actinobacillus pleuropneumoniae, Porcine circovirus* type 2 (PCV2) caused infections and atrophic rhinitis caused by toxigenic *Pasteurella multocida.* In many countries, vaccines against diseases such as salmonellosis, Porcine Reproductive and Respiratory Syndrome (PRRS) and Transmissible Gastroenteritis (TGE) are also used depending on commercial availability.

A further example of such a vaccine is one for active immunization against oedema disease caused by a bacterial toxin known as Shigatoxin 2e (Stx2e) that is produced by certain strains of *E. coli.* Oedema disease leads to fluids accumulating in the tissues of the stomach and gut. Damage to the wall of small blood vessels in the brain results in characteristic nervous signs. Oedema disease is found worldwide and usually occurs in the first two weeks after weaning of the piglets and is an acute (short-term) condition which can lead to death within 24 to 48 hours. A commercially available vaccine against oedema disease is ECOPORC® SHIGA produced by IDT Biologika. ECOPORC® SHIGA is a vaccine containing a form of Shigatoxin (Stx2e) that has been genetically modified so that it can no longer cause the disease. The vaccine is given as a single injection into a muscle, preferably the neck muscle behind the ear. Protection starts 21 days after vaccination and lasts for 105 days. ECOPORC® SHIGA contains aluminium hydroxide as an adjuvant and thus qualifies as an adsorbed vaccine.

Iron is an essential building block for humans and animals. As it is well-known, it is responsible for the formation of haemoglobin necessary for the transport of oxygen throughout the body. Many other essential proteins and enzymes in the body also require adequate levels of iron. Therefore, additional iron helps farm animals such as piglets to grow strong and to stay healthy.

Farm animals, such as piglets, are especially susceptible to iron deficiency for several reasons. For example, since modern pig breeding practices keep sows and piglets indoors in concrete pants that offer no chance for the animals to acquire iron from soil, an iron deficiency might occur. Furthermore, the sow's milk is low in iron. The milk contributes only 1 mg iron per day; however, the actual need of a piglet is approximately 7 to 16 mg per day for proper growth and health (National Research Council (1998): Nutrient Requirements of Swine, 10th revised edition, Washington D.C.: National Academy Press.). Furthermore, piglets have an extremely high growth rate compared to other mammals requiring a great amount of iron as well.

Therefore, iron deficiency anaemia in piglets is usually prevented by administration of iron shortly after birth (between day 1 and 3). The administration of iron preparations may occur parenterally and usually includes iron (III) dextran complexes or iron dextran-heptonic acid complexes (gleptoferron) as active ingredient. Gleptoferron may be injected or administered perorally.

The livestock sector globally is highly dynamic. In developing countries, it is evolving in response to rapidly increasing demand for livestock products, whereas in developed countries, demand for livestock products is stagnating while many production systems are increasing their efficiency.

The cost pressure involved in farm animal growth is high and thus usually only one single injection is acceptable from an economic perspective (an injection of an iron-based preparation). As a consequence, preventive vaccination (i.e. a second injection of a vaccine) is not accomplished and, rather, treatment of the animals with antibiotics is done in practice (which is deemed to be more cost-effective).

Based on these circumstances and additionally considering the animal welfare aspect, there is an ongoing need to improve vaccines, for example by designing new combined vaccines which can be administered by one single injection step only.

For a vaccine/vaccine-combination, a number of scientific-technical solutions are known, e.g. combinations against *Porcine circovirus* type 2 (PCV2) and *Mycoplasma hyopneumoniae*; against different serotypes of *E. coli* or against *Bordetella bronchiseptica* and *Pasteurella multocida.*

However, vaccine/pharmaceutical-combinations have not become viable as yet. Incompatibilities between the active ingredients and/or excipients on the one hand and antigens and/or adjuvants on the other are one reason therefore. For example, incompatibilities between aluminium hydroxide (used as adjuvant) and iron complexes have been reported.

### SUMMARY OF THE INVENTION

It is therefore an object underlying the invention to provide a combined preparation for parenteral use suitable for a single administration to a farm animal, such as a piglet. It is a further object of the present invention to provide a combined preparation for parenteral use having a prophylactic and/or a therapeutic effect as good as that of two separate injections and low side effects at the same time. It is a still further object of the invention to provide a combined preparation fulfilling the high quality criteria of pharmaceutical industry.

These objects are achieved by the present invention.

According to one aspect of the present invention, a combined preparation for parenteral use is provided comprising the following components:
a) an adsorbed vaccine, and
b) complexed iron in a physiologically acceptable form.

The combined preparation might be present as a mixture or a kit-of-parts of components a) and b).

Surprisingly, it turned out that such a combined preparation is stable at least for a defined period of time so that it can be provided as a mixture or as a kit-of-parts. In the latter, the components are mixed prior to administration of the overall preparation to a farm animal.

After having performed intensive studies, the inventors found that a combined preparation of an adsorbed vaccine and complexed iron is stable, has similar effectiveness as the separate components and does not cause any side effects in excess to those of the individual components administered separately. It was found that the mixed preparation has a sufficient stability for a time period of between about 0 to 7 days.

These findings are surprising since one would expect interactions after combining components a) and b). According to the entry in DrugBank (www.drugbank.ca), iron dextran can be used for the treatment of patients with documented iron deficiency. In chapter "Interactions", there is an explicit warning in view of drug interactions with the adjuvant aluminium hydroxide. Aluminium hydroxide is used as an adjuvant in many adsorbed vaccines. Aluminium hydroxide is said to cause a decrease in the absorption of iron dextran resulting in a reduced serum concentration and potentially a decrease in efficacy.

Furthermore, it is also known in the field of pharmaceutical products that oral iron supplements might interact with antacids containing aluminium hydroxide. It is noted that those antacids may bind to iron supplements thus preventing them from being solved.

These findings are surprising since based on the composition of the components intra-mixture interactions between the adjuvant of vaccine (aluminium hydroxide) and gleptoferron can be expected. Both the adsorption of gleptoferron on aluminium hydroxide and the complexation of aluminium ions by dextran glucoheptonic acid, the complexing agent of gleptoferron, are relevant.

It turned out that the extent of negative impact caused by said interactions can be limited when an adsorbed vaccine and complexed iron are mixed (i.e. are in physical contact) for a limited time span of up to 7 days, preferably up to about 24 hours. The inventors showed in the Examples (see below) that combined preparations of adsorbed vaccines and iron complexes resulted in an immune protection of the animals vaccinated which is not significantly different from the immune protection conferred by the adsorbed vaccine alone (i.e. without addition of iron complexes). See in particular chapters "Summary", "Results" and "Conclusions" of the attached Examples.

Furthermore, it could be shown that, as a result of the biochemical analysis of blood samples received from test animals, the iron levels in animals treated with a combined preparation of the present invention are highly similar to that of animals which have been exclusively injected with an iron preparation. This finding is unexpected since the known interactions suggest a lower iron level after application of a combined preparation as after application of iron complexes (such as gleptoferron) alone. It is referred to the attached Examples, chapter "Results of the URSOFERRAN® 200 mg/ml pro inj. application": The haemoglobin values (g/l) on study day 14 after vaccination are comparable between the animals of groups 1, 2 (combined vaccine) and 4 (gleptoferron only) - irrespective of the time point of mixing (group 1 and 2). The results of the blood investigation are entirely unexpected since one would assume a lower haemoglobin level in the test samples taken from the animals of group 1 and 2. However, as it can be seen from the table in 1.1.5., the hemoglobin levels are similar between groups 1, 2 and 4 (and are notably slightly higher in groups 1 and 2 vs. group 4: 116 and 112 g/l vs. 111 g/l).

### DETAILED DESCRIPTION OF THE INVENTION

According to a first aspect, the present invention is directed to a combined preparation for parenteral use comprising the following components:
a) an adsorbed vaccine, and
b) complexed iron in a physiologically acceptable form, where each component optionally contains one or more pharmaceutically acceptable auxiliaries.

By definition, the term "combined preparation" (or "combined vaccine") means that the preparation is in the form of a mixture of components a) and b) or in the form of a kit-of-parts comprising components a) and b) in spatially distinct form. In the latter case, the combined preparation is admixed in advance of the administration of the preparation as it will be explained later.

According to the *Guideline on the requirements for combined vaccines and associations of immunological veterinary medicinal products* (IVMPs) issued by the European Medicines Agency, a combined vaccine is an IVMP intended for immunization against more than one disease and/or pathogen and which is authorized by one marketing authorization. The combined vaccine can be supplied in a single primary container or in several primary containers, the contents of which are mixed prior to use for administration.

One component of the combined preparation of the present invention is an adsorbed vaccine. An adsorbed vaccine, by definition, is a vaccine where the immunologically active ingredient (such as the immunizing antigen) is present bound to an adsorbent, also called adjuvant. These adsorbing adjuvants usually are selected from aluminium hydroxide, aluminium phosphate or calcium phosphate. Basically, the function of the adjuvants is to slow down the resorption of the antigens and, therefore, improving the immune reaction.

Among the adjuvants generally used in an adsorbed vaccine, aluminium adjuvants, in particular aluminium hydroxide, is preferred. Aluminium adjuvants are the most widely used adjuvants in both human and veterinary vaccines. These adjuvants have been used in practical vaccination for more than 60 years and are generally recognized as safe and as stimulators of immunity.

The second component of the combined preparation of the invention is complexed iron in a physiologically acceptable form. "Physiologically acceptable form" as used herein means that the complexed iron can be resorbed by the human or animal body thereby treating or preventing a potential iron deficiency. Furthermore, this term means that the complexed iron does not cause any adverse effects and is well-tolerated by the human or animal body. The complexed iron usually is present in the form of iron(III) ions in complexed form, for example as iron(III) dextran complex or, iron(III) hydroxide-dextran-glucoheptonic acid complex.

The INN name of the iron(III) hydroxide-dextran-glucoheptonic acid complex is gleptoferron (it is a macromolecular complex of iron(III) hydroxide and dextran glucoheptonic acid). Injectable gleptoferron as, for example, a 10, 15 or 20 % by weight aqueous solution is used for the prevention and treatment of iron deficiency or anaemia in farm animals such as piglets. For example, it can be used in an amount of 1 ml of 20 % by weight gleptoferron solution by intramuscular injection during the first three days of life. The amount of the complexed iron administered can be chosen according to the standard knowledge in the field as it is available for years.
The combined preparation for parenteral use according to the present invention further optionally contains one or more pharmaceutically acceptable auxiliaries.

The term "auxiliaries" as used herein, comprises all further additives, liquids, etc. which are generally used in preparations for parenteral use. For example, auxiliaries include water for injection (WFI) prepared according to the requirements of the USP or the European Pharmacopoeia. Further auxiliaries added may include antimicrobial agents as required (such as phenylmercuric nitrate and thiomersal, benzalkonium chloride, phenol, etc.). Further additives for parenteral preparations generally might include substances as defined by the USP as those to maintain or safeguard the quality of the parenteral preparation. For example, substances may be added to increase or to maintain drug solubility such as complexing agents and surface active agents. For example, cyclodextrins may be named as complexing agents or polyoxyethylene sorbitan monolaurate (claim 20), etc. may be named as a surface active agent.

Furthermore, substances may be added to make the parenteral solution isotonic or to provide near physiological pH. Common tonicity adjusters are sodium chloride, dextrose and glycerine.

Furthermore, means to enhance the chemical stability of the solution such as antioxidants, inert gases, chelating agents and buffers may be added.

Other auxiliaries may include sustaining and/or controlling drug release agents such as polymers, suspending agents, emulsifying agents, etc. Regarding further information on how to produce parenteral preparations and the standards applied to them, it is referred to Remington, the Science and Practice of Pharmacy, 22nd edition, 2013, published by Pharmaceutical Press. In addition, the respective chapters of the USP and European Pharmacopoeia can be taken as a reference.

In one preferred embodiment, the combined preparation of the present invention is selected from the group of pig vaccines against oedema disease caused by Shigatoxin Stx2e producing *E. coli* (STEC), *Mycoplasma hyopneumoniae, influenza, PCV*2, *PRRSV, Streptococcus suis, Actinobacillus pleuropneumonia, Rhinitis atrophicans, Haemophilus parasuis, Erysipelas,* classical swine fever and *Brachyspira hyodysenteriae.*

As noted above, when the combined preparation for parenteral use is in the form of a kit-of-parts, it comprises two separate containers, such as ampoules or vials. That is to say, the injectable formulations of the present invention are packaged into containers made of glass or plastic. Container systems include ampoules and vials as mentioned above, and additionally syringes, cartridges, bottles and bags. A skilled person knows how to select the precise container (and the suitable closure) according to pharmaceutical standard considerations such as the intended use, time of storage, storage stability, etc. Regarding details on containers and closures, it is generally referred to Remington, 22nd edition, 2013, pages 870-898.

A particularly preferred embodiment of the present invention is directed to a combined preparation as defined above, where component a) is a vaccine against oedema disease caused by Shigatoxin Stx2e producing *E. coli,* which vaccine is containing aluminium hydroxide as an adjuvant, and a component b) is an aqueous solution of gleptoferron.

According to a second aspect, the preparation of the present invention may be used for the immunization of farm animals, such as piglets, where the immunization comprises the steps of
a) providing a mixture according to one or more of the aspects and embodiments defined above; or
b) providing a kit-of-parts as defined herein above, and mixing the ingredients of each component in order to obtain a ready-to-use preparation;
c) administering the preparation to farm animals, such as piglets.

Although the present invention is described mainly focussed on the use of the combined preparation in piglets, it is evident that the combined preparation has a broad application in the field of veterinary science and is not restricted to piglets. Therefore, for example, the combined preparation can be used for vaccination of other livestock, such as domesticated animals raised in an agricultural setting such as cattle, donkey, goat, sheep, rabbits, and many others.

According to the present invention, the mixing in step b) is performed in a time span of o hours to 7 days prior to administration of the preparation to the animal in need thereof. Preferably, the mixing in step b) is performed 0.5 hours to 24 hours, more preferably 1 hour to 6 hours prior to administration of the preparation.

As noted above, the immunization is, for example, against oedema disease caused by Shigatoxin Stx2e producing E. coli, etc.

The present invention now will be explained by the enclosed examples. However, it should be clear that the present invention is not restricted to the contents of the examples which are merely illustrative.

### EXAMPLES

### Onset of immunity after the application of the combined preparation of the vaccine ECOPORC® SHIGA and the iron-dextran complex URSOFERRAN® 200 mg/ml pro inj. (challenge at the age of 25 days)

Evaluation of the efficacy of the combined preparation of the vaccine ECOPORC® SHIGA and the iron-dextran complex URSOFERRAN® 200 mg/ml pro inj. in suckling piglets at the minimum age of 4 days after the administration of the combined single dose.

ECOPORC® SHIGA is a commercially available vaccine produced and marketed by IDT Biologika, Dessau-Rosslau, Germany. Details on the vaccine are available from EMA at http://www.ema.europa.eu/ema/index.jsp?curl=pages/medicines/veterinary/medicines/ 002588/vet_ed_000270.jsp&mid=WC0b01ac058008d7a8

URSOFERRAN® 200 mg/ml pro inj. contains gleptoferron as active ingredient and is marketed by Serumwerke Bernburg AG, Bernburg, Germany.

### Scope and aim of the trial

The objective of this study was to test the efficacy of vaccine ECOPORC® SHIGA after the combined application together with URSOFERRAN® 200 mg/ml pro inj. Therefore the combined application of ECOPORC® SHIGA with URSOFERRAN® 200 mg/ml pro inj. was done at the recommended age and route: suckling piglets at the age of 4 days and by using the intramuscular route. The challenge by using a special developed Stx2e-model was performed at the age of 25 days, corresponding to the onset of immunity (OOI).

88 suckling piglets at the age of 4 days were used in the test.

Twenty two suckling piglets were used for testing the combination of ECOPORC® SHIGA with URSOFERRAN® 200 mg/ml pro inj. mixed immediately prior to vaccination, twenty two suckling piglets were used for testing the combination of ECOPORC® SHIGA with URSOFERRAN® 200 mg/ml pro inj. mixed six hours prior to vaccination, twenty two suckling piglets were used for testing the vaccine ECOPORC® SHIGA alone and twenty two suckling piglets were used for testing URSOFERRAN® 200 mg/ml pro inj. alone. The piglets of this group were also used as the challenge control piglets.

The parameters used to evaluate the efficacy of the combined application of ECOPORC® SHIGA with URSOFERRAN® 200 mg/ml pro inj. and to evaluate the OOI were:
1. number of surviving/dead animals after the administration of the challenge material 21 days after immunisation and the effects on general health affection (typical symptoms of edema disease)
2. evaluation of the efficacy of the application of URSOFERRAN® 200 mg/ml pro inj on the basis of blood samples taken on study day 0, 14 and 35 (biochemical tests).

### Reference to EP monographs

- European Pharmacopoeia Monograph 8.0, 5.2.7. Evaluation of Efficacy of Vaccines and Immune Sera for veterinary use
- EMA/CVMP/IWP/206555/2010, Guideline on requirements for the production and control of immunological veterinary medicinal products (14 June 2012)

### Summary

It was demonstrated after the challenge given 21 days after application of the preparations that an application of a combined preparation of ECOPORC® SHIGA and URSOFERRAN® 200 mg/ml pro inj. using the recommended application method in the target species/category did not result in differences regarding the number of surviving animals, compared to the status of ECOPORC® SHIGA administered alone. These observations were irrespective of the time the two VMPs were mixed.

Furthermore no effect on the haemoglobin content was observed in animals that were treated with URSOFERRAN® 200 mg/ml pro inj. This was irrespective of the time of mixing the two VMPs or from the time point of application of URSOFERRAN® 200 mg/ml pro inj. Only group 3 (animals without a URSOFERRAN® 200 mg/ml pro inj. application) was affected as described below: the group showed lower results detected by the biochemical blood samples investigation

### Animals, materials and methods

| | |
|---|---|
| | **88 total** |
| **Animals:** | |
| **Vaccination groups:** | **group 1:** |
| | **22 animals** |
| | **(combination* of ECOPORC® SHIGA with URSOFERRAN® 200 mg/ml pro inj. (mixed 0 hours prior to vaccination))** |
| | **group 2:** |
| | **22 animals** |
| | **(combination* of ECOPORC® SHIGA with URSOFERRAN® 200 mg/ml pro inj. (mixed 6 hours prior to vaccination))** |
| | **group 3:** |
| | **22 animals** |
| | **(ECOPORC® SHIGA)** |
| | |
| | ***... this means "mixing"** |
| | |
| **Control groups:** | **group 4:** |
| | **22 animals** |
| | **(URSOFERRAN® 200 mg/ml pro inj. + Stx2e-challenge)** |
| | |
| **Materials:** | |
| **Test substance 1:** | **ECOPORC® SHIGA** |
| | |
| **Test substance 2:** | **URSOFERRAN® 200 mg/ml pro inj.** |
| | |
| **Challenge material:** | **Specific Stx2e toxin** |

### Methods:

| | |
|---|---|
| **Vaccination** | |
| **Dosing schedule** | **groups 1, 2:** |
| | **2.0 ml per animal i.m. in the *Regio auricularis*** |
| | **once, 4^{th} day of life** |
| | |
| | **group 3:** |
| | **1.0 ml per animal i.m. in the *Regio auricularis*** |
| | **once, 4^{th} day of life** |
| | |
| | **group 4:** |
| | **1.0 ml per animal i.m. in the *Regio auricularis*** |
| | **once, 4^{th} day of life** |
| | |
| | |
| **Challenge** | |
| **Challenge route** | **intravenous** |
| | |
| | |
| **Challenge time point** | **25^{th} day of life** |
| | |
| | |
| | |
| **Biochemical investigation** | **- blood sampling of surviving animals on study days 0, 14 and 35** |

### Evaluation:

| | |
|---|---|
| **Determination of protected animals/ unprotected animals:** | **- evaluation of surviving/dead animals** |
| | |
| | |
| **Determination of haemoglobin content** | **- evaluation of the test results** |

### Results

### Results of the Stx-2e challenge:

### Evaluation of surviving/dead animals

| | **vaccination: study day 0** | **surviving/dead animals** |
|---|---|---|
| group1 | ECOPORC® SHIGA + Fe, mixed 0 h | 15/7 |
| | | (68.20 %) |
| group 2 | ECOPORC® SHIGA + Fe, mixed 6 h prior to application mixed | 12/9* |
| | | (57.10%) |
| group 3 | ECOPORC® SHIGA | 14/8 |
| | | (63.60%) |
| group 4 | URSOFERRAN® 200 mg/ml pro inj. | 0/22 |
| | | (0.00 %) |

| | | |
|---|---|---|
| *...one piglet was not challenged | | |

### Results of the URSOFERRAN® 200 mg/ml pro inj. application:

| **study day** | **group** | **haemoglobin g/l** |
|---|---|---|
| 0 (before application) | 1 | 78 |
| | 2 | 73 |
| | 3 | 84 |
| | 4 | 86 |
| 14 (before challenge) | 1 | 116 |
| | 2 | 112 |
| | 3 | 44 |
| | 4 | 111 |
| 35 (14 days after challenge) | 1 | 99 |
| | 2 | 99 |
| | 3 | 47 |
| | 4 | - |

### Summary of results

The application of a combined preparation of the vaccine ECOPORC® SHIGA and URSOFERRAN® 200 mg/ml pro inj. (mixed 0 hours and 6 h prior to application) to the target animals under the recommended conditions for use demonstrates the efficacy under well-controlled laboratory conditions after the challenge with the Stx2e. The components for the combined preparations were commercial batches, the ECOPORC® SHIGA batch had an efficacy of 4.3 x 10⁶ CD_{50equivalent}/ml after a shelf life of more than two years.

The following items were observed:
A) Results relating to combination of ECOPORC® SHIGA with URSOFERRAN® 200 mg/ml pro inj. (in comparison to the animals of group 4): After the challenge a significantly higher number of surviving animals vaccinated with the combination - irrespective of the time point of mixing (groups 1 and 2) and the single application of ECOPORC® SHIGA alone (group 3)
B) Results related to URSOFERRAN 200 mg/ml pro inj.:
   ∘ The results of the biochemical examinations are comparable within the animals of the groups 1 to 4 before the application of URSOFERRAN® 200 mg/ml pro inj. on study day 0
   ∘ On study day 14 the results are comparable between the animals of groups 1, 2, and 4 - irrespective of the time point of mixing (group 1 and 2). The results of the blood investigation of the animals of group 3 (single application of ECOPORC® SHIGA) are significantly lower than the results of the animals of groups 1, 2, and 4. On study day 35 the results are comparable between the animals of groups 1 and 2 - irrespective of the time point of mixing. The results of the blood investigation of the animals of group 3 (single application of ECOPORC® SHIGA) are significantly lower than the results of the animals of groups 1 and 2.

The onset of immunity against a severe Stx2e-challenge is demonstrated in animals on the challenge day 21 days after the stand-alone immunisation with ECOPORC® SHIGA and in combined application with URSOFERRAN® 200 mg/ml pro inj., irrespective of the time of mixing prior to application. During the course of the study, one animal (group 3) was excluded for reasons unrelated to the vaccine or vaccination.

### Conclusion

The application of the combined preparations of ECOPORC® SHIGA and URSOFERRAN® 200 mg/ml pro inj., irrespective of the time of mixing prior to application, protects animals as soon as 21 days after the intramuscular application of one dose at the age of 4 days. The protection ratio of the animals vaccinated with ECOPORC® SHIGA both in combination and as the stand-alone immunisation (groups 1, 2, 3) was significantly higher than the animals of group 4.

The differences between the animals of the groups 1, 2 and 3 are not significant.

The results of the biochemical investigation of the blood samples of the animals of groups 1, 2, 4 are significantly higher than the results of the animals in group 3.

The differences between groups 1, 2 and 4 were not significant.

## Claims

1. A combined preparation for parenteral use comprising the following components:
a) an adsorbed vaccine, and
b) complexed iron in a physiologically acceptable form,
each component optionally containing one or more pharmaceutically acceptable auxiliaries.

2. The combined preparation of claim 1, which is in the form of a mixture or a kit-of-parts of components a) and b).

3. The combined preparation of one or more of the preceding claims, where iron is present as iron (III), preferably in complexed form.

4. The combined preparation of claim 3, where iron is present as iron (III) hydroxide-dextran-glucoheptonic acid complex (gleptoferron) or as an iron (III)-hydroxide dextran complex.

5. The combined preparation of one or more of the preceding claims, where the vaccine is selected from the group of pig vaccines against Edema Disease caused by Shigatoxin Stx2e-producing *E*. *coli* (STEC), *Mycoplasma hyopneumoniae, Influenza,* PCV2, PRRSV, Streptococcus *suis, Actinobacillus pleuropneumonia, Rhinitis atrophicans, Haemophilus parasuis, Erysipelas,* Classical swine fever and *Brachyspira hyodysenteriae.*

6. The combined preparation of one or more of the preceding claims, where the combination is in the form of a kit-of-parts comprising two separate containers, such as ampoules or vials.

7. The combined preparation of one or more of the preceding claims, where component a) is recombinant toxin vaccine against Edema Disease caused by Shigatoxin Stx2e-producing E. coli (STEC) containing aluminium hydroxide as an adjuvant, and component b) is an aqueous solution of gleptoferron.

8. A combined preparation for parenteral use of one or more of the preceding claims, for use in the immunization of piglets, where the immunization comprises the steps of:
a) providing a mixture according to one or more of claims 1-7; or
b) providing a kit-of-parts according to one or more of claims 1-7 and mixing the ingredients of each component in order to obtain a ready-to-use-preparation;
c) administering the preparation to a piglet.

9. The combined preparation of claim 8, where the mixing in step b) is performed 0 h - 7 days prior to administration of the preparation.

10. The combined preparation of claim 8, where the mixing in step b) is performed 0.5 h - 24 h, preferably 1 h - 6 h prior to administration of the preparation.

11. The combined preparation of one or more of claims 8-10, where the immunization is against Edema Disease caused by Shigatoxin Stx2e-producing *E. coli* (STEC), *Mycoplasma hyopneumoniae, Influenza,* PCV2, PRRSV, *Streptococcus suis, Actinobacillus pleuropneumonia, Rhinitis atrophicans, Haemophilus parasuis, Erysipelas,* Classical swine fever and *Brachyspira hyodysenteriae.*
